Europäisches Patentamt

European Patent Office

Office européen des brevets

(11) Publication number: **0 433 765 A1**

# EUROPEAN PATENT APPLICATION

(21) Application number: **90123297.5**

(22) Date of filing: **05.12.90**

(51) Int. Cl.⁵: **A61K 37/24**, A61K 37/36, A61K 37/66, A61K 31/675, A61K 31/557

(30) Priority: **18.12.89 IT 2272589**

(43) Date of publication of application: **26.06.91 Bulletin 91/26**

(84) Designated Contracting States: **AT BE CH DE DK ES FR GB GR LI LU NL SE**

(71) Applicant: **CONSIGLIO NAZIONALE DELLE RICERCHE**
**Piazzale Aldo Moro, 7**
**I-00195 Roma(IT)**

(72) Inventor: **Favalli, Cartesio**
**Corso Matteoti, 208**
**I-04100 Latina(IT)**
Inventor: **Garaci, Enrico**
**Via Salaria, 237**
**I-00198 Roma(IT)**

(74) Representative: **Gervasi, Gemma et al**
**NOTARBARTOLO & GERVASI Srl Viale Bianca Maria 33**
**I-20122 Milan(IT)**

(54) **The use of immunomodulants as synergistic agents of chemotherapeutics in tumor therapy.**

(57) The present invention refers to the use of immunomodulants as synergistic agents of chemotherapeutics useful in tumor therapy.

EP 0 433 765 A1

# THE USE OF IMMUNOMODULANTS AS SYNERGISTIC AGENTS OF CHEMOTHERAPEUTICS IN TUMOR THERAPY

## State of the Art

In the last few years, increasing attention has been given to the potential applications of biological answer modifiers in tumor therapy. These substances, the most important of which are the interferons, the interleukins, the tumor necrosis factor, the thymus hormones and the colony stimulator factor, stimulating the immunitary system and in particular certain cellular effectors which can induce in tumoral cells cytostatic and/or cytolytic effects, have opened up a new front in the battle against tumors. At present, however, while immunotherapy has demonstrated in numerous experimental models a considerable effectiveness, the clinical results have deluded, being at any rate below expectations. One should consider, for instance, the limits in the clinical utilization in man imposed by the loss of stimulating NK (Natural Killer) activity of interferons, in immunodepressed patients, or by the toxicity of interleukin-2.

In preceeding studies we have put in evidence the fact that thymic hormones, prostaglandins and interferon, when administered alone, are ineffective in reviving the NK activity. When, on the other hand, the interferon administration is preceded by a treatment with thymic hormones or with prostaglandins, a synergistic effect is obtained and the interferon stimulating activity is revived. Similar results are obtained when employing interleukin-2 in place of interferon.

In this case the synergistic effect of thymic hormones and of prostaglandins allows to lower the interleukin doses thus lowering the toxicity.

However, the use of thymic hormones and of prostaglandins as synergistic agents of interferon and interleukin-2, although having brought about a certain advance in the field of anti tumor therapy, has failed to produce results which may be considered as definitely acceptable. See on this point:

CELLULAR IMMUNOLOGY 106 43-52 Academic Press. Inc. 1987;

CANCER IMMUNOL. IMMUNOTHER. 20 189-92 Springer-Verlag 1985;

RECENT ADVANCES IN AUTOIMMUNITY AND TUMOR IMMUNOLOGY, 211-217.

Franco Dammacco, Edi-Ermes. Milan 1988:

ADVANCES IN IMMUNOMODULATION, 169-79 Pythagora-Press, Roma-Milan 1988.

## Detailed description of the invention

In conclusione, nothing has been found up to now which may allow to use with satisfactory results, in patients affected by tumors, interferons also in immunodepressive situations (which are normal in patients of this type) or to use interleukin-2 in therapeutically effective and non toxic doses.

We now have surprisingly found, and this is an object of the present invention, that immunomodulants selected from the group consisting of thymus hormones and prostaglandins, in combination with immunomodulants selected in the groups consisting of interferons and interleukin-2, show an unexpected synergistic effect with the chemotherapeutics employed in curing tumoral affections, so that the chemotherapeutic effect is substantially potentiabed, leading in many cases to final positive results.

It is therefore an object of the present invention the use of immunomodulants as synergistic agents of the chemotherapeutics known in the therapy of tumoral affections.

Particularly useful chemoterapeutics in the context of the present invention are the following: Cyclophosphamide, 5-Fluorouracyl, Carmustin, Adriamicin, Decarbazine, Cisplatin.

### Experimental Section

Experimental studies on C57BL/6NcrLBR mice

Tumors used: Lewis Lung carcinoma (3LL) and B-16 Melanoma

Chemotherapeutics employed: Cyclophosphamide (200 mg/Kg)

5-Fluoruracyl (120 mg/Kg), Carmustin (20 mg/Kg), Adriamicin (7,5 mg/Kg), Decarbazin (50 mg/Kg) and Cisplatin (16 mg/Kg).

Immunomodulants employed: Thymic hormone (Thymosin $\alpha1$, 200 g/Kg for 4 days), Interferon (natural $\alpha$-$\beta$ murine, Interferon, 30,000 I.U./mouse in one administration), human recombined Interleukin-2 (1,000 I.U./mouse in one administration).

## Treatment outline

The animals were subcutaneously inoculated with 2 x $10^5$ hormonal cells on day 0. On day 8, when all tumors were palpable, the animals were divided at random in 12 groups and treated respectively with:
  A) Control diluent
  B) Chemotherapeutic (day 8)
  C) Chemotherapeutic (day 8) and immunotherapy with thymus hormone (days 12 and 13)
  D) Chemotherapeutic (day 8) and immunotherapy with interferon (day 13)
  E) Chemotherapeutic (day 8) and immunotherapy with thymosin (days 10 and 13) and interferon (day 13)
  F) Chemotherapeutic (day 8) and immunotherapy with interleukin-2 (day 13)
  G) Chemotherapeutic (day 8) and immunotherapy with tymosin (days 10-13) and interleukin-2 (day 13)
  H) Immunotherapy with thymosin (days 10-13)
  I) Immunotherapy with interferon (day 13)
  L) Immunotherapy with IL-2 (day 13)
  M) Immunotherapy with thymosin (days 10-13) and interferon (day 13)
  N) Immunotherapy with thymosin (days 10-13) and interleukin-2 (day 13)

## Results

| Group | Treatment | | | |
|---|---|---|---|---|
| | α Thymosin or Thymopentin | Interferon | Interleukin-2 | Average Survival Time + Stand.dev. |
| 1 | - | - | - | 24.4 + 2.0 |
| 2 | + | - | - | 27.7 + 1.9 |
| 3 | - | + | - | 28.1 + 1.8 |
| 4 | - | - | + | 26.8 + 1.5 |
| 5 | + | + | - | 31.9 + 1.6 |
| 6 | + | - | + | 30.8 + 1.9 |

The experimental data above show that the immunotherapy alone, obtained with the single imunomodulant agents, does not substantially modify the tumor growth dyanmics, while the one obtained with thymosin-interferon or thymosin-interleukin-2 association slows down the growth of both B-16 melanoma and 3LL , protracting the average survival times of the treated animals. In no case, however, does the immunotherapy alone show a curative effect, as all the animals die in the end.

The treatment with the chemotherapeutic alone according to the treatment protocols previously described, does, on the other hand, in the most favourable case, as in the treatment of 3LL with cyclophosphamide, cure 10% of the mice.

The association of chemotherapy with only one immunomodulant such as the association of cyclophosphamide with thymus hormone or with interleukin-2, succeeds in curing in the first case 35% and in the second 30% of the animals.

The results obtained associating combined immunoterapy with chemotherapy have amply demonstrated that the combined treatment with thymus hormone and interferon or thymus hormone and interleukin-2 increases in a very significant way the effects of chemotherapy.

In the case of 3LL treated with cyclophosphamide, for instance, in comparison with the animals treated only with chemotherapy, the ones treated also with the thymosin-interferon combination have shown a 75% survival.

For the ones treated, after chemotherapy, with the thymosin-interleukin-2 combination, the survival was 60%. It should be noted that the synergistic effect of the combined immunotherapy is correlated with the specific activity of the chemotherapeutic against the particular type of tumor. Thus, as said, cyclophosphamide together with combined immunotherapy constitutes the most efficient treatment against 3LL, because this tumor is relatively sensitive to this chemotherapeutic, while in the case of B-16 melanoma the most efficient treatment proved to be decarbazine followed by combined immunotherapy.

Clinical evaluation of the results obtained on experimental animal models.

Transferring to humans the treatments performed on animals is favoured by the modest toxicity of interferon at the intended dosages (2,000,000 I.U./mg $\alpha$ or $\beta$ interferon) while no side effects are expected in the use of thymus hormones.

The combined immunotherapy protocol may be inserted between chemotherapy cycles without modifying, as far as possible, their timing.

We have started a study on patients with an advanced stage of colon-rectal carcinoma, who could not be treated radically by surgery, and having measurable neoplastic lesions.

The protocal provides:

Folinic acid 200 mg/sm on days 1, 2, 3, 4, 5;

Chemotherapy with 5-Fluorouracyl 400 mg/sm 15 minutes after the folinic acid administration, diluted in 100 ml physiological saline infused in 30 minutes.

The patients are then divided in two groups, one of which is treated by combined immunotherapy, the other with placebo. The immunotherapy consists in a treatment with thymus hormone (thymosin $\alpha$ 1, 1 mg sc, or Tp5, 50 mg sc) on days 8, 9, 10, 11, and $\alpha$ interferon (2 millions I.U. sc) on day 12.

The cycles are repeated every 28 days.

The results obtained by us allow to conclude that the immunotherapeutic treatment notably improves the chemotherapeutic treatment, considering both immunological and clinical parameters (decrease of tumoral lesions measurable by echography and abdominal ACT, reduction of CEA in the positives, prolongation of survival times).

The results of our research work on experimental animal models lead us to evaluate as highly effective also an immunotherapeutic treatment to be associated with chemotherapy with thymus hormones and interleukin.

In this case it is possible to use low dosages of this last substance which is known to be very active in high dosages in stimulating effector cells having anti-tumor activity, and, which, however, also possesses a high toxicity and is therefore scarcely usable.

The pre-treatment with thymic hormone allows in fact to utilize in mice interleukin-2 doses ca. 100 times lower (100-1000 U/kg/h in slow infusion) than the ones employed at present, while obtaining the same stimulation of cell populations having cytotoxic activity. Table I reports the limit chemotherapeutic and immunomodulant dosages and the respective administration modes.

<div align="center">

TABLE I

</div>

| Chemotherapeutic | Dosage | Administration |
|---|---|---|
| Cyclophosphamide | 500-1500 mg/m$^2$ | i.v. |
| 5-Fluorouracyl | 800-1200 mg/m$^2$ | i.v. |
| Carmustin | 150-250 mg/m$^2$ | i.v. |
| Adriamicin | 45-90 mg/m$^2$ | i.v. |
| Decarbazin | 300-400 mg/m$^2$ | i.v. |
| Immunomodulant | Dosage | Administration |
| Tymus hormones | 0.5-50 mg/m$^2$ | s.c. |
| Interferons | 0.5-2 x 10$^6$ U./m$^2$ | i.m. |
| Interleukin-2 | 1-10 x 10$^5$ U./m$^2$/h | slow infusion |
| Prostaglandine | 3-30 mg/m$^2$ | i.v. |

In conclusion, it was experimentally proved that the use of immunomodulants, particularly in associations such as thymus hormones and interferon or thymus hormones and interleukin-2, in succession to the use of any known chemotherapeutic, has a strong as well as unforeseen and inexplicable effect on the activity of the chemotherapeutic in the therapy of tumors.

**Claims**

1. The use of immunomodulants as synergistic agents of chemotherapeutics for the treatment of tumor diseases.

2. The use according to claim 1 in which the immunomodulants are selected from the group consisting of: thymus hormones and prostaglandins in combination with immunomodulants selected from the group consisting of interferon and interleukin-2.

3. The use according to claim 2 wherein the thymic hormone is $\alpha$ 1 thymosin or thymopentin.

4. The use according to claim 1 wherein the chemotherapeutics are selected from the group consisting of: Cyclophosphamide, 5-Fluoruracyl , Carmustin , Adriamicin , Decarbazin and Cisplatin.

5. The use according to claim 2 in which the immunomodulants are thymus hormones and interferon.

6. The use according to claim 5 in which the immunomodulants are $\alpha$ 1 thymosin or thymopentin and interferon.

7. The use according to claim 2 in which the immunomodulants are: thymus hormones and interleukin-2.

8. The use according to claim 2 in which the immunomodulants are thymosin and interleukin-2.

9. The use according to claim 2 in which the immunomodulants are prostaglandins and interferon.

10. The use according to claim 2 in which the immunomodulants are prostaglandins and interleukin-2.

11. The use according to claim 1 in which the chemotherapeutic employed is cyclophosphamide and the immunomodulants are thymus hormones and interferon.

12. The use according to claim 11 in which the thymus hormone is $\alpha$-thymosin.

13. The use according to claim 1 in which the chemotherapeutic employed is cyclophosphamide and the immunomodulants are thymus hormones and interleukin-2.

14. The use according to claim 13 in which the thymus hormone employed is $\alpha$ 1 thymosin.

15. The use according to claim 1 in which the chemotherapeutic employed is decarbazin and the immunomodulants are thymus hormones and interferon.

16. The use according to claim 15, in which the thymus hormone is $\alpha$ 1 thymosin.

European
Patent Office

EUROPEAN SEARCH
REPORT

Application Number

EP 90 12 3297

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | DIALOG 07198977 MEDLINE abstr. 90105977 E. GARACI et al. :"Enhanced immune response and antitumor immunity with combinations of biologic al response modifiers." & Bull N Y Acad Med, Jan 1989, Vol. 65(1), p.: 111-119 * abstract * | 1-8,11-14 | A 61 K 37/24 A 61 K 37/36 A 61 K 37/66 A 61 K 31/675 A 61 K 31/557 |
| X | DI-A-6 019 2   (EXERPTA MEDICA)86014228 FAVALLI C. et al.: "Modulation of natural killer activity by thymosin alpha 1 and interferon" & CANCER IMMUNOL. IMMUNOTHER., vol. 20/3, 1985 p.: 189-192 * abstract * | 1-6,11,12 | |
| X | DI-A-5 298 2   (EXERPTA MEDICA)83049336 THEILEN G.H.; HILLS D.: "Comparative aspects of cancer immunotherapy. Immunologic methods used for treatment of spontaneous cancer in animals" & J. AM. VET. MED. ASSOC., vol. 181/10, 1982 p.: 1134-1141 | 1 | |
| X | DI-A-5 608 8   (EXERPTA MEDICA)84104482 FUDENBERG H.H.; WHITTEN H.D.: "Immunostimulation : Synthetic and biological modulators of immunity" & ANNU. REV. PHARMACOL. TOXICOL., vol. 24,1984, p.:147-174 * abstract * | 1 | TECHNICAL FIELDS SEARCHED (Int. Cl.5) A 61 K |
| X,P | WO-A-8 902 734   (MATRIX PHARMACEUTICALS, INC.) * claims 1, 2, 4 * | 1,4 | |
| X | EP-A-0 385 859   (CERIANI, ROBERTO LUIS) * abstract * | 1 | |

The present search report has been drawn up for all claims

| Place of search | Date of completion of search | Examiner |
|---|---|---|
| The Hague | 21 March 91 | LEHERTE C.F.M. |

CATEGORY OF CITED DOCUMENTS

X : particularly relevant if taken alone
Y : particularly relevant if combined with another
  document of the same catagory
A : technological background
O : non-written disclosure
P : intermediate document
T : theory or principle underlying the invention

E : earlier patent document, but published on, or after
  the filing date
D : document cited in the application
L : document cited for other reasons

&: member of the same patent family, corresponding
  document